# EUROPEAN PATENT SPECIFICATION

(11) **EP 2 399 612 B1**
(45) Date of publication and mention of the grant of the patent: **21.02.2018**
(21) Application number: 10838989.1
(22) Date of filing: 24.12.2010
(51) Int. Cl.: A61K 49/00, A61K 51/00, G01N 33/15, G01N 33/50, G01N 33/58

(54) **COMPOSITION FOR LABELING TISSUES OF CENTRAL NERVOUS SYSTEM, METHOD FOR LABELING TISSUES OF CENTRAL NERVOUS SYSTEM, AND SCREENING METHOD USING THE COMPOSITION FOR LABELING TISSUES OF CENTRAL NERVOUS SYSTEM**
ZUSAMMENSETZUNG ZUR GEWEBEKENNZEICHNUNG IM ZENTRALNERVENSYSTEM, VERFAHREN ZUR GEWEBEKENNZEICHNUNG IM ZENTRALNERVENSYSTEM UND SCREENING-VERFAHREN MIT DER ZUSAMMENSETZUNG ZUR GEWEBEKENNZEICHNUNG IM ZENTRALNERVENSYSTEM
COMPOSITION POUR MARQUER DES TISSUS DU SYSTÈME NERVEUX CENTRAL, PROCÉDÉ POUR MARQUER DES TISSUS DU SYSTÈME NERVEUX CENTRAL, ET PROCÉDÉ DE CRIBLAGE UTILISANT LA COMPOSITION POUR MARQUER DES TISSUS DU SYSTÈME NERVEUX CENTRAL

(30) Priority: 25.12.2009 JP 2009296270
(43) Date of publication of application: 28.12.2011
(73) Proprietor: Canon Kabushiki Kaisha, Tokyo 146-8501 (JP)
(72) Inventor: WATANABE, Kohei, Tokyo 146-8501 (JP); SHINTOU, Taichi, Tokyo 146-8501 (JP); NOMOTO, Tsuyoshi, Tokyo 146-8501 (JP); MIYAZAKI, Takeshi, Tokyo 146-8501 (JP); OKANO, Mie, Tokyo 146-8501 (JP); TANAKA, Toshio, Tsu-shi Mie 514-8507 (JP); NISHIMURA, Yuhei, Tsu-shi Mie 514-8507 (JP); SHIMADA, Yasuhito, Tsu-shi Mie 514-8507 (JP)
(74) Representative: WESER & Kollegen
(86) International application number: PCT/JP2010/007519
(87) International publication number: WO 2011/077751

(56) References cited:
- JP-A- 53 095 619
- JP-A- 2000 344 684
- JP-A- 2003 502 321
- JP-A- 2004 250 411
- JP-A- 2004 292 571
- JP-A- 2005 332 589
- JP-A- 2007 106 755
- JP-A- 2010 148 447
- JP-A- 2010 168 369
- JP-A- 2010 229 353
- US-A- 4 582 779
- DHIRAJ G KABRA ET AL: "Neuroprotective effect of 4-amino-1,8-napthalimide, a poly(ADP ribose) polymerase inhibitor in middle cerebral artery occlusion-induced focal cerebral ischemia in rat", BRAIN RESEARCH BULLETIN., vol. 62, no. 5, 1 February 2004 (2004-02-01), pages 425-433, XP55277080, GB ISSN: 0361-9230, DOI: 10.1016/j.brainresbull.2003.11.001
- LIU Y ET AL: "Novel fluorescent markers for hypoxic cells of naphthalimides with two heterocyclic side chains for bioreductive binding", BIOORGANIC & MEDICINAL CHEMISTRY, PERGAMON, GB, vol. 14, no. 9, 1 May 2006 (2006-05-01), pages 2935-2941, XP027992554, ISSN: 0968-0896 [retrieved on 2006-05-01]
- ISABELLE BERQUE-BESTEL ET AL: "Synthesis and Characterization of the First Fluorescent Antagonists for Human 5-HT 4 Receptors", JOURNAL OF MEDICINAL CHEMISTRY, vol. 46, no. 13, 1 June 2003 (2003-06-01), pages 2606-2620, XP55277078, US ISSN: 0022-2623, DOI: 10.1021/jm0307887
- PIECHOWSKI A P ET AL: "Desirable Properties of Photovoltaic Dyes", JOURNAL OF PHYSICAL CHEMISTRY, AMERICAN CHEMICAL SOCIETY, US, vol. 88, no. 5, 1 January 1984 (1984-01-01), pages 934-950, XP002464705, ISSN: 0022-3654, DOI: 10.1021/J150649A020
- ANLIKER, R. ET AL.: 'Bioaccumulation of dyestuffs and organic pigments in fish. Relationships to hydrophobicity and steric factors' CHEMOSPHERE vol. 17, no. 8, 1987, pages 1631 - 44, XP055094258

## Description

### Technical Field

The present invention relates to a labeling composition capable of clearly labeling a central nervous system tissue, a method for labeling a central nervous system tissue using the central nervous system tissue-labeling composition, and a screening method using the central nervous system tissue-labeling composition.

### Background Art

Recently, a number of patients with central nervous system diseases has been on the increase along with the aging of society. Representative examples of the diseases include Parkinson's disease, Alzheimer's disease, epilepsy, migraine, spinocerebellar degeneration, brain tumor, cerebral hemorrhage, and cerebral infarction. Central nervous system diseases often impair motor function and cognitive function, leading to a significant decrease in a patient's quality of life. Thus, it is desired that an abnormality in the central nervous system tissue be accurately recognized for early detection of a disease so that therapy or a measure to retard the progression is provided. For diagnosis in the central nervous system tissue, morphological assessments by imaging using computer tomography (CT) and magnetic resonance imaging (MRI), and radionuclide imaging diagnoses by a method such as positron emission tomography (PET) are employed.

The brain is tissues containing neurons and glial cells. The brain performs advanced functions through complex intercellular networks and hierarchical structures. An imaging technology allows visualized measurement without impairing the function of the central nervous system, enabling more intuitive as well as dynamic and quantitative examination. Recently, development of not only the aforementioned imaging diagnosis techniques but also new techniques such as fluorescent imaging and near-infrared imaging is ongoing.

For imaging of the central nervous system, methods of using various probes to add contrast to the tissue for visualization of a pathological site(s) are developed. For example, in PET and single photon emission computed tomography (SPECT), a method is employed in which a synthetic compound labeled with a radioactive isotope (ligand) is administered to the body, and then localized radioactivities in the brain are quantitated to analyze the distribution of the ligand in the body and the metabolic dynamics of the ligand for mapping of the functional localization. Also, a compound that specifically binds to β-amyloid which is deposited in the brain tissue and that is used for diagnosis of Alzheimer's disease is disclosed (Patent Literatures 1 and 2). Because these compounds (probes) administered to the living body label β-amyloid present in the brain, a site where these probes are markedly accumulated can be detected by a PET apparatus. Besides the above, a compound capable of fluorescently labeling glial cells in the brain is disclosed in Non-Patent Literature 1. These probes with properties of accumulating in and labeling the central nervous system as described above are utilized, in clinical setting, for the diagnosis by visualization of a disease state, and also, in a basic research, as a tool for the mechanism analysis of a disease. Non-Patent Literature 3 discloses 4-amino-1,8-napthalimide, a poly(ADP ribose) polymerase inhibitor in middle cerebral artery occlusion-induced focal cerebral ischemia in rat. Non-Patent Literature 4 discloses fluorescent markers for hypoxic cells of naphthalimides with two heterocyclic side chains of 2-nitroimidazole for bioreductive binding. Non-Patent Literature 5 discloses the synthesis and characterization of fluorescent antagonists for human 5-HT₄ receptors.

### Citation List

### Patent Literature

- PTL 1:: Japanese Patent Application Laid-Open No. 2004-250411
- PTL 2:: Japanese Patent Application Laid-Open No. 2007-106755
- PTL 3:: U.S. Patent Application Publication No. 2006/0193776

### Non Patent Literature

- NPL 1:: Nature Methods., 1(1), pages 31-37 (2004)
- NPL 2:: Brain Research Bulletin 75, pages 619-628 (2008)
- NPL 3:: D. Kabra et al., Brain Research Bulletin 62, pages 425-433 (2004)
- NPL 4:: Y. Liu et al., Bioorganic & Medicinal Chemistry, vol. 14, pages 2935-2941 (2006)
- NPL 5:: I. Berque-Bestel, J. Med. Chem., vol. 46, pages 2606-2620 (2003)

### Summary of Invention

### Technical Problem

The conventionally-employed probes utilize their properties such as accumulating in a site where a large amount of glucose is taken up or of specifically accumulating to β-amyloid; therefore, they are not suitable for morphological imaging of a specific site in the brain in diseases not associated with such a specific site. Also, there is a problem with the aforementioned glial cell-staining compound because the compound requires such a highly invasive treatment that involves direct administration to the brain.

In the first place, the transferability of a compound to the brain is regulated by the blood-brain barrier (BBB) and the blood-cerebrospinal fluid barrier (BCSFB), and many of the compounds that can migrate into a normal tissue may not be able to migrate into the brain. As described in the aforementioned Patent Literature 3, some of a compound capable of migrating into the brain in the juvenile stage loses its transferability to the brain once BBB is functioning.

Further, although the aforementioned Patent Literatures 1 and 2 report a BBB-permeable coumarin compound, the technologies disclosed therein only focus on the compound's specific binding ability to β-amyloid, while these literatures are silent on a property of labeling the central nervous system tissue in the brain.

In view of the above, a labeling compound for the central nervous system tissue capable of clearly labeling the central nervous system tissue of the living body alive without being affected by BBB and BCSFB is demanded.

### Solution to Problem

The present inventors conducted an intensive study to solve the aforementioned problems pertaining to the conventional technology. As a result, they have found that dye compounds represented by the following general formula (1) are capable of labeling the central nervous system tissue of the living body alive. That is, they have found that the compounds label at least any one of the tissues including optic nerve, optic tract, superior colliculus (optic tectum), pituitary gland, tectospinal (tectobulbar) tract, and reticular formation with high sensitivity, serving as a novel central nervous system tissue-labeling compound as defined in claim 1, enabling highly accurate diagnosis and screening of a drug, thereby completing the present invention.

Also, the present inventors have established a method for labeling the central nervous system tissue of the living body as defined in claim 5. Further, the present inventors have developed a screening method using a labeling composition of the present invention as defined in claim 6, thereby completing the present invention. The other claims relate to further developments.

Specifically, the novel compound for the central nervous system tissue of the present invention is defined in claim 1, and is able to label at least any one of the tissues including optic nerve, optic tract, superior colliculus (optic tectum), pituitary gland, tectospinal (tectobulbar) tract, and reticular formation.

### Advantageous Effects of Invention

Provision of the central nervous system tissue-labeling composition of the present invention has enabled selective labeling of a brain tissue such as optic nerve, optic tract, superior colliculus (optic tectum), pituitary gland, tectospinal (tectobulbar) tract, and reticular formation, which has been conventionally difficult. This enables simple and highly precise assessment and analysis of the morphology and the state of cells of a specific site in central nervous system tissues. Further, a screening method using the central nervous system tissue-labeling composition of the present invention can be a novel, effective tool for the research and the discovery of a drug for the central nervous system.

### Brief Description of Drawings

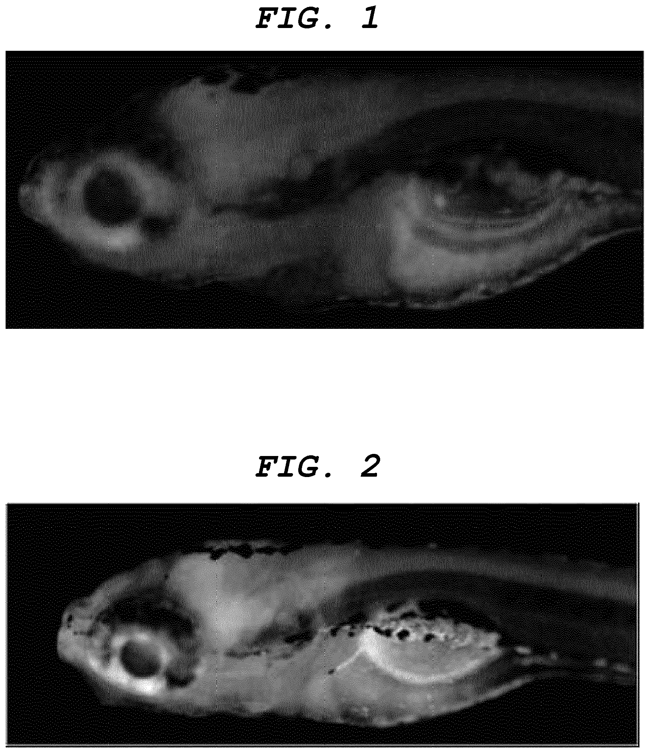

[Fig. 1]Fig. 1 is an image of the labeled central nervous system tissues observed in Example 2.
[Fig. 2]Fig. 2 is an observational image of the central nervous system tissues observed in Example 5.

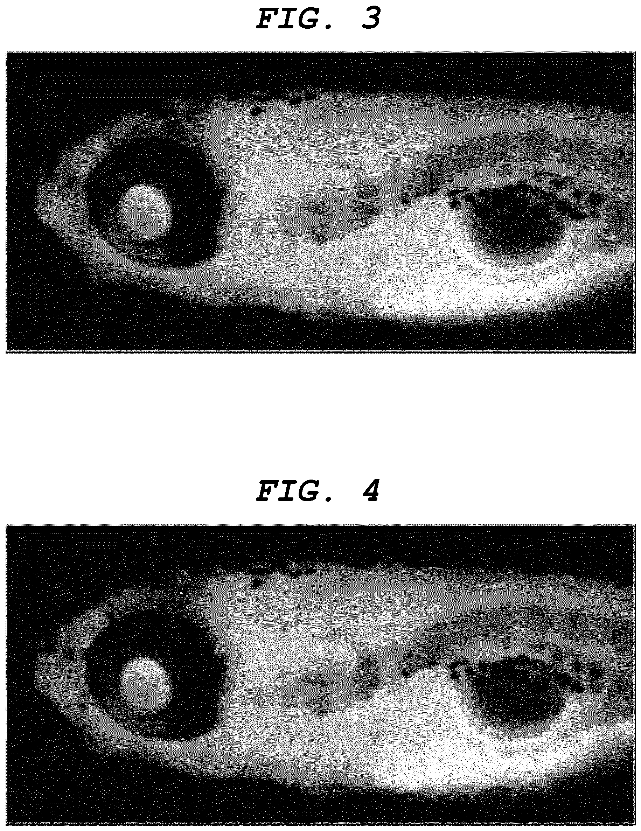

[Fig. 3]Fig. 3 is an observational image of the central nervous system tissues observed in Example 6.
[Fig. 4]Fig. 4 is a confocal microscopic image of the central nervous system tissues observed in Example 2.

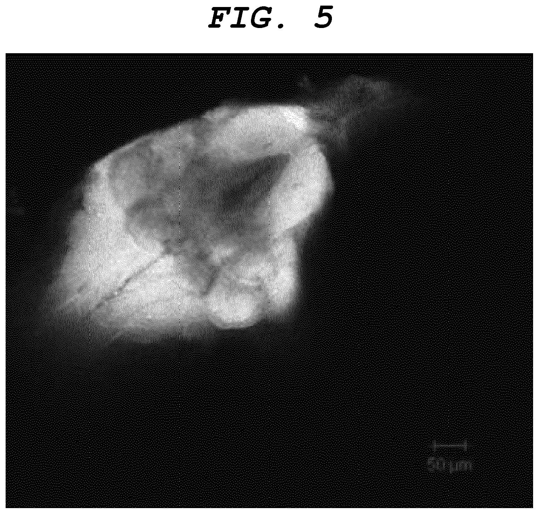

[Fig. 5]Fig. 5 is a confocal microscopic image of the central nervous system tissues observed in Example 3.

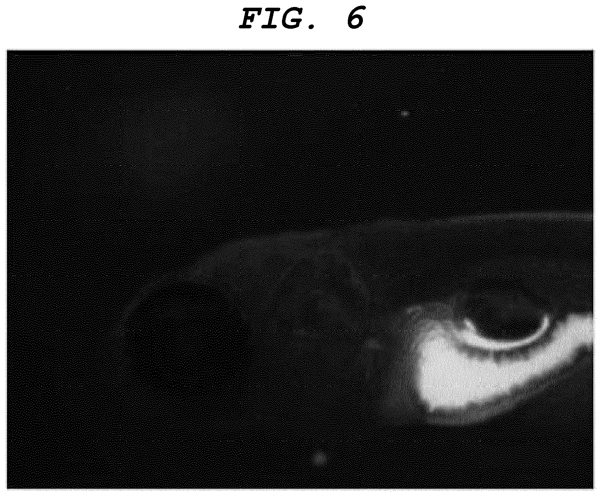

[Fig. 6]Fig. 6 is an observational image of zebrafish observed in Comparative Example 1.

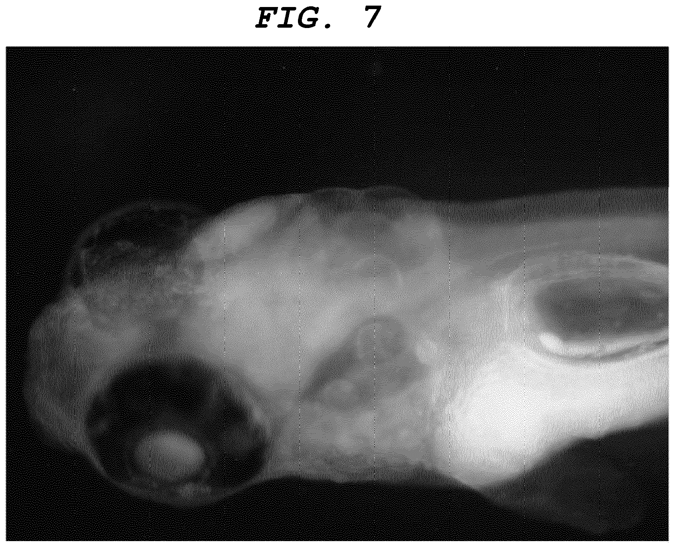

[Fig. 7]Fig. 7 is an observational image of the central nervous system tissues of a day-14 embryo of zebrafish observed in Example 8.

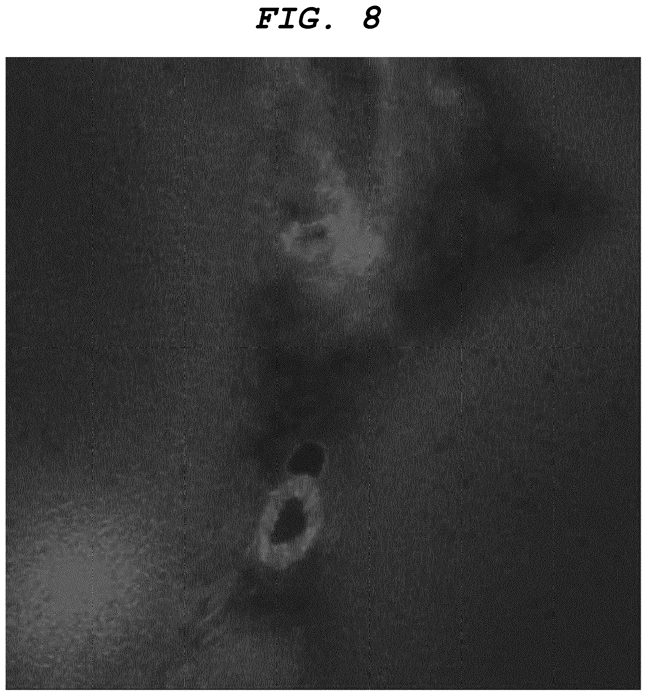

[Fig. 8]Fig. 8 is a confocal microscopic image of a section of the mouse central nervous system tissues observed in Example 10.

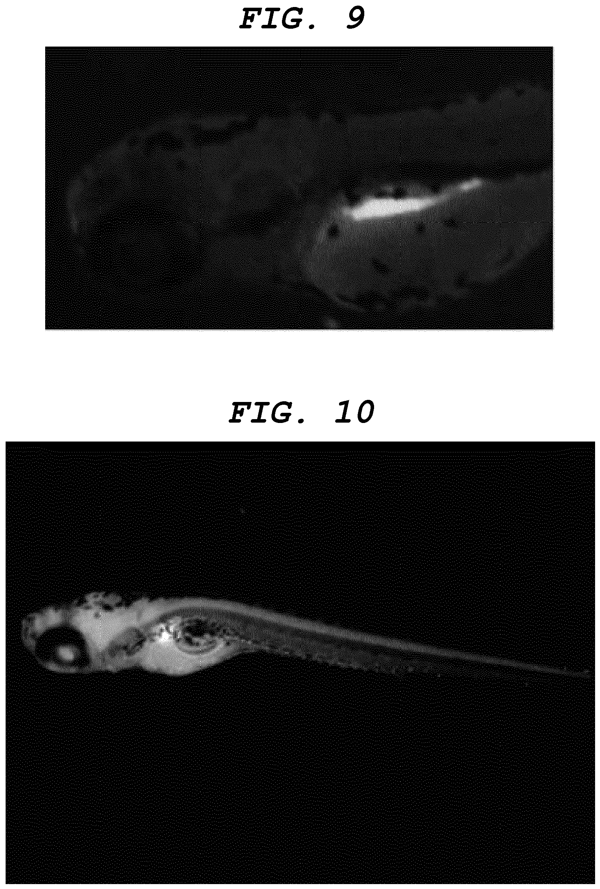

[Fig. 9]Fig. 9 is an observational image of zebrafish observed in Reference Example 1.
[Fig. 10] Fig. 10 is an image of the labeled central nervous system tissues observed in Example 12.

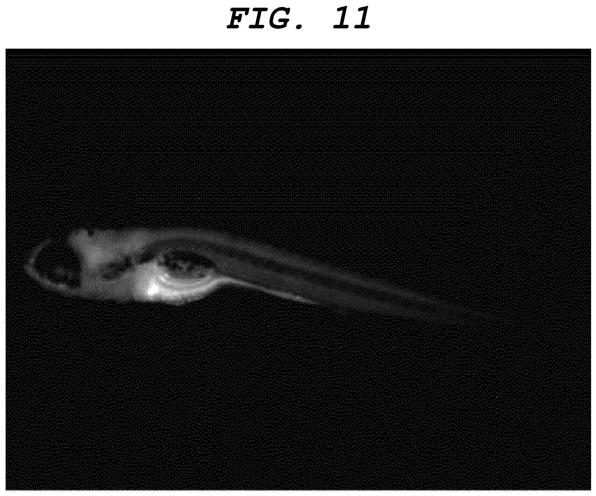

[Fig. 11]Fig. 11 is an image of the labeled central nervous system tissues observed in Example 15.

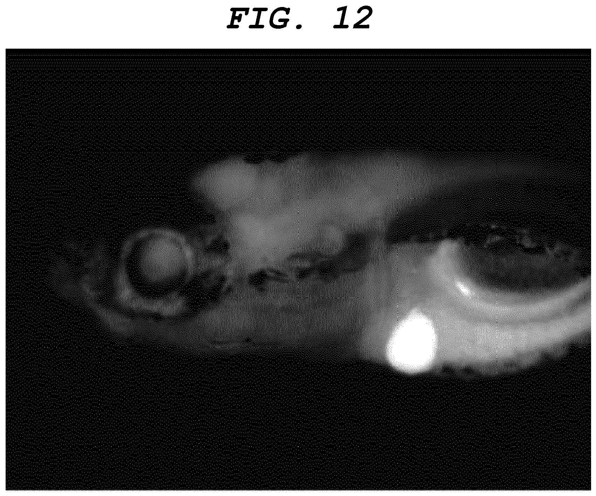

[Fig. 12]Fig. 12 is an observational image of the central nervous system tissues observed in Example 16.

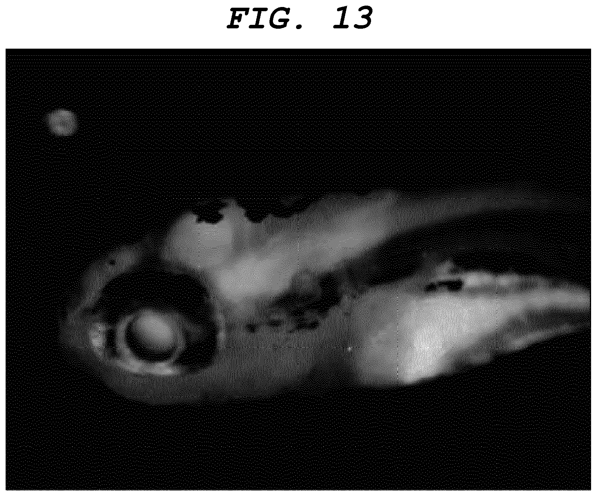

[Fig. 13]Fig. 13 is an observational image of the central nervous system tissues observed in Example 20.

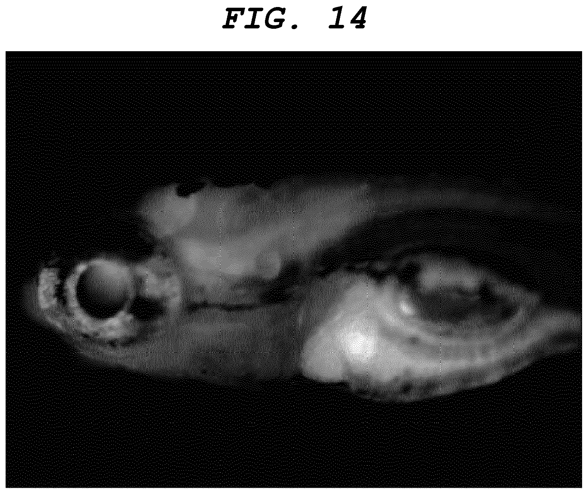

[Fig. 14]Fig. 14 is an observational image of the central nervous system tissues observed in Example 24.

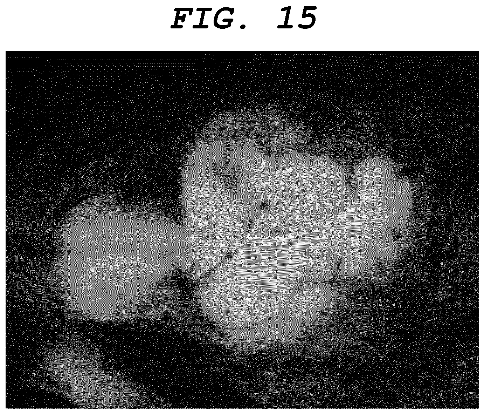

[Fig. 15]Fig. 15 is an observational image of the central nervous system tissues of three-month-old zebrafish observed in Example 26.

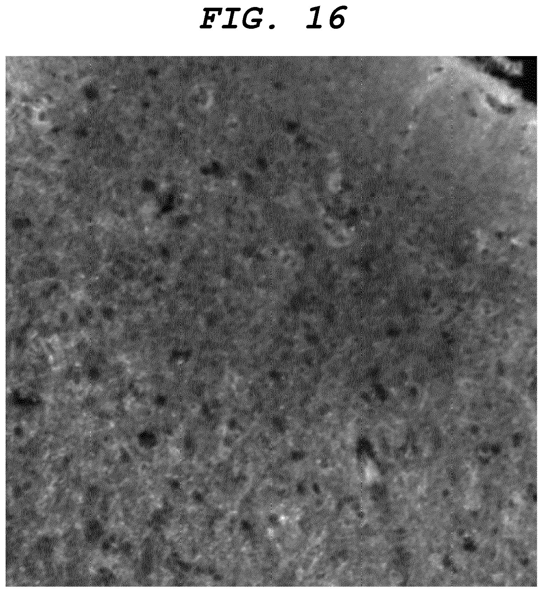

[Fig. 16]Fig. 16 is a confocal microscopic image of a section of the mouse central nervous system tissues observed in Example 27.

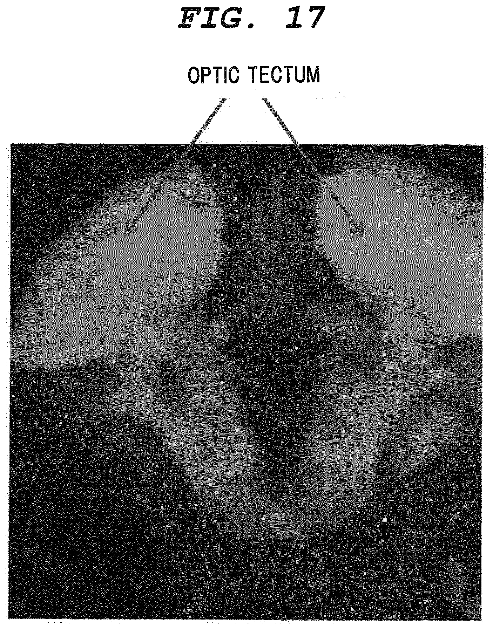

[Fig. 17]Fig. 17 is a coronal cross-sectional view of the brain of juvenile zebrafish observed in Example 29, illustrating an image of labeled optic tectum.

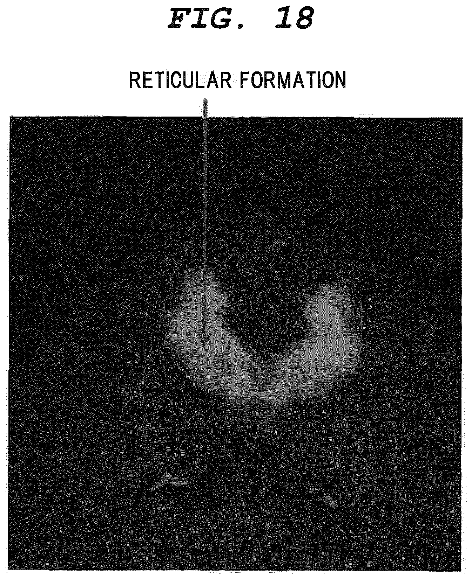

[Fig. 18]Fig. 18 is a coronal cross-sectional view of the brain of juvenile zebrafish observed in Example 29, illustrating an image of labeled reticular formation.

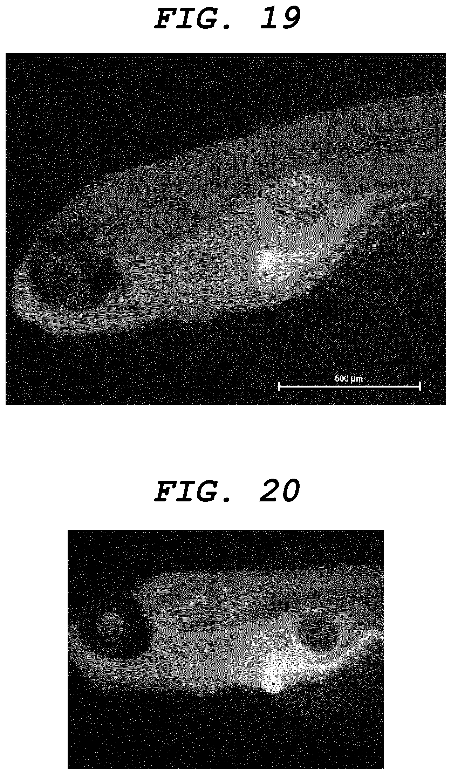

[Fig. 19]Fig. 19 is an observational image of the central nervous system tissues observed in Example 33.
[Fig. 20]Fig. 20 is an observational image of the central nervous system tissues observed in Example 42.

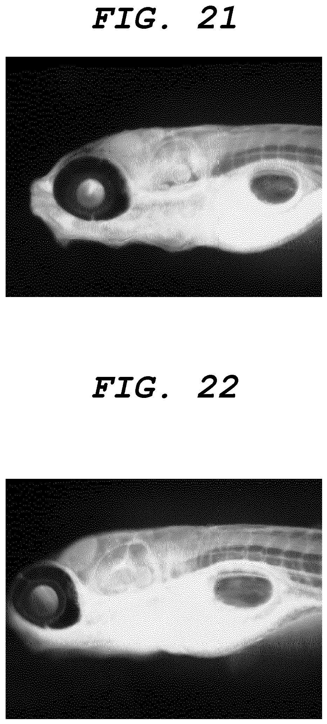

[Fig. 21]Fig. 21 is an observational image of the central nervous system tissues observed in Example 43.
[Fig. 22]Fig. 22 is an observational image of the central nervous system tissues observed in Example 45.

### Description of Embodiments

Hereinbelow, the embodiments of the present invention will be described with reference to drawings. It is to be noted that the embodiments to be individually disclosed below are examples of the central nervous system tissue-labeling composition, the method for labeling the central nervous system tissue, and the screening method using the central nervous system tissue-labeling composition according to the present invention, and the present invention is not limited to these examples.

### First embodiment

The central nervous system tissue-labeling composition according to a first embodiment of the present invention is characterized by containing, as an active ingredient, at least one of compounds represented by the general formula (1) wherein aromatic ring A represents a structure of the following formula (2).

Further compounds (not according to the invention) are characterised by the structures (1) wherein the aromatic ring A is a structure of one of formulae (3) or (4), or wherein aromatic ring A represents, through binding to R2 via N, a structure represented by formulae (5) or (6), or the structure (7).

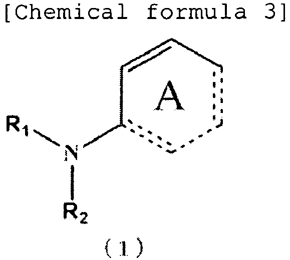

In the general formula (1), R₁ to R₂ each independently represent a hydrogen atom, an alkyl group, an aralkyl group, or an aryl group. Also, an aromatic ring A represents a skeletal structure represented by the following general formulas (2) to (4), or an aromatic ring A represents, through binding to R₂ via N, a skeletal structure represented by the following general formulas (5) to (6):

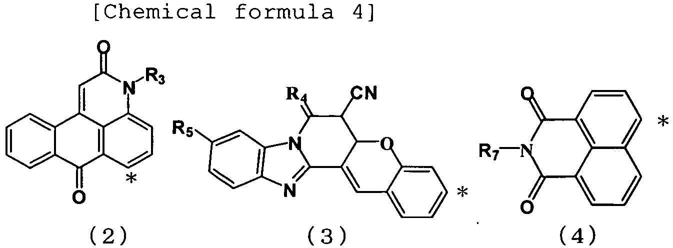

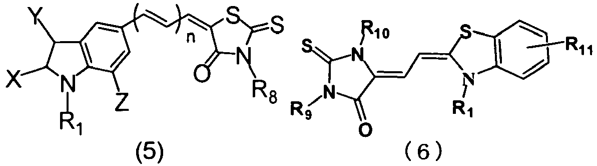

In the general formula (2), R₃ represents a hydrogen atom, an alkyl group, an aralkyl group, or an aryl group. In the general formula (3), R₄ represents an oxygen atom, a sulfur atom, or N(R₆), R₅ represents a hydrogen atom, an alkyl group, an alkoxy group, or a sulfonic acid group, and R₆ represents a hydrogen atom, an alkyl group, or an aryl group. In the general formula (4), R₇ represents a hydrogen atom, an alkyl group, an aryl group, or a heterocyclic group. It is to be noted that, in the general formulas (2), (3), and (4), '*' represents a binding site to N in the general formula (1). In the general formula (5), R₈ represents an alkyl group or an alkyl chain having a carboxy group at its end, X and Y represent a hydrogen atom or an alkyl group, Z represents a hydrogen atom or a halogen atom, and n represents an integer of 0 or 1. Alternatively, X and Y may be bound together to form a ring. In the general formula (6), R₉ to R₁₀ represent an alkyl group or an aryl group, and R₁₁ represents a hydrogen atom, an alkyl group, an alkoxy group, a carboxylic acid group, or a sulfonic acid group.

No particular limitation is imposed on the alkyl group at R₁ to R₂ in the aforementioned general formula (1), and examples thereof include a methyl group, an ethyl group, a propyl group, a butyl group, a cyclohexyl group, and a 3-hexanyl group. Also, the alkyl group may further contain a substituent as long as the substituent does not markedly deteriorate the preservation stability of the dye compound. No particular limitation is imposed on the aralkyl group at R₁ to R₂, and examples thereof include a benzyl group and a phenethyl group. Also, the aralkyl group may contain a substituent. Also, no particular limitation is imposed on the aryl group at R₁ to R₂, and examples thereof include a phenyl group and a naphthyl group. Also, the aryl group may contain a substituent.

No particular limitation is imposed on the alkyl group at R₃ in the aforementioned general formula (2), and examples thereof include a methyl group, an ethyl group, a propyl group, a butyl group, and a cyclohexyl group. In the compound represented by the aforementioned general formulas (1) and (2), particularly, when one of R₁ and R₂ is a hydrogen atom and the other is an alkyl group or an aralkyl group, intense fluorescence is obtained. Thus, such a compound can be employed. R₃ can be a methyl group, a butyl group, and a cyclohexyl group for easiness of synthesis.

No particular limitation is imposed on the alkyl group at R₅ to R₆ in the aforementioned general formula (3), and examples thereof include a methyl group, an ethyl group, a propyl group, and a butyl group. No particular limitation is imposed on the alkoxy group at R₅, and examples thereof include a methoxy group, an ethoxy group, a propoxy group, and a butoxy group. No particular limitation is imposed on the aryl group at R₆, and examples thereof include a phenyl group.

No particular limitation is imposed on the alkyl group at R₇ in the aforementioned general formula (4), and examples thereof include a methyl group, an ethyl group, a propyl group, and a butyl group. No particular limitation is imposed on the aryl group at R₇, and examples thereof include a phenyl group. Also, the aryl group may further contain a substituent as long as the substituent does not markedly deteriorate the preservation stability of the dye compound. No particular limitation is imposed on the heterocyclic group at R₇, and examples thereof include a pyridyl group, a pyrazyl group, and a morpholinyl group.

No particular limitation is imposed on the alkyl group at R₈, X, and Y in the aforementioned general formula (5), and examples thereof include a methyl group, an ethyl group, a propyl group, and a butyl group. Also, the alkyl group may further contain a substituent as long as the substituent does not markedly deteriorate the preservation stability of the dye compound. No particular limitation is imposed on the halogen atom at Z in the aforementioned general formula (5), and examples thereof include a fluorine atom, a chlorine atom, a bromine atom, or an iodine atom. No particular limitation is imposed on the ring formed by binding of X and Y in the aforementioned general formula (5), and examples thereof include a cyclopentane ring and a benzene ring. No particular limitation is imposed on the alkyl group at R₉ to R₁₀ in the aforementioned general formula (6), and examples thereof include a methyl group, an ethyl group, a propyl group, and a butyl group. No particular limitation is imposed on the aryl group at R₉ to R₁₀, and examples thereof include a phenyl group. Also, the aryl group may further contain a substituent as long as the substituent does not markedly deteriorate the preservation stability of the dye compound.

No particular limitation is imposed on the alkyl group at R₁₁, and examples thereof include a methyl group, an ethyl group, a propyl group, and a butyl group. No particular limitation is imposed on the alkoxy group at R₁₁, and examples thereof include a methoxy group, an ethoxy group, a propoxy group, and a butoxy group. While the dye compound represented by the general formulas (1) to (6) is commercially obtainable, it can also be synthesized in accordance with a publicly known method.

Preferable specific examples of the dye compound represented by the general formulas (1) to (6) (compounds (8) to (14) or compounds (29) to (45)) will be shown below; compounds (8), (9), (29), (30), (31), (32) and (33) are according to present claim 1. The further compounds are not in the scope of the claims.

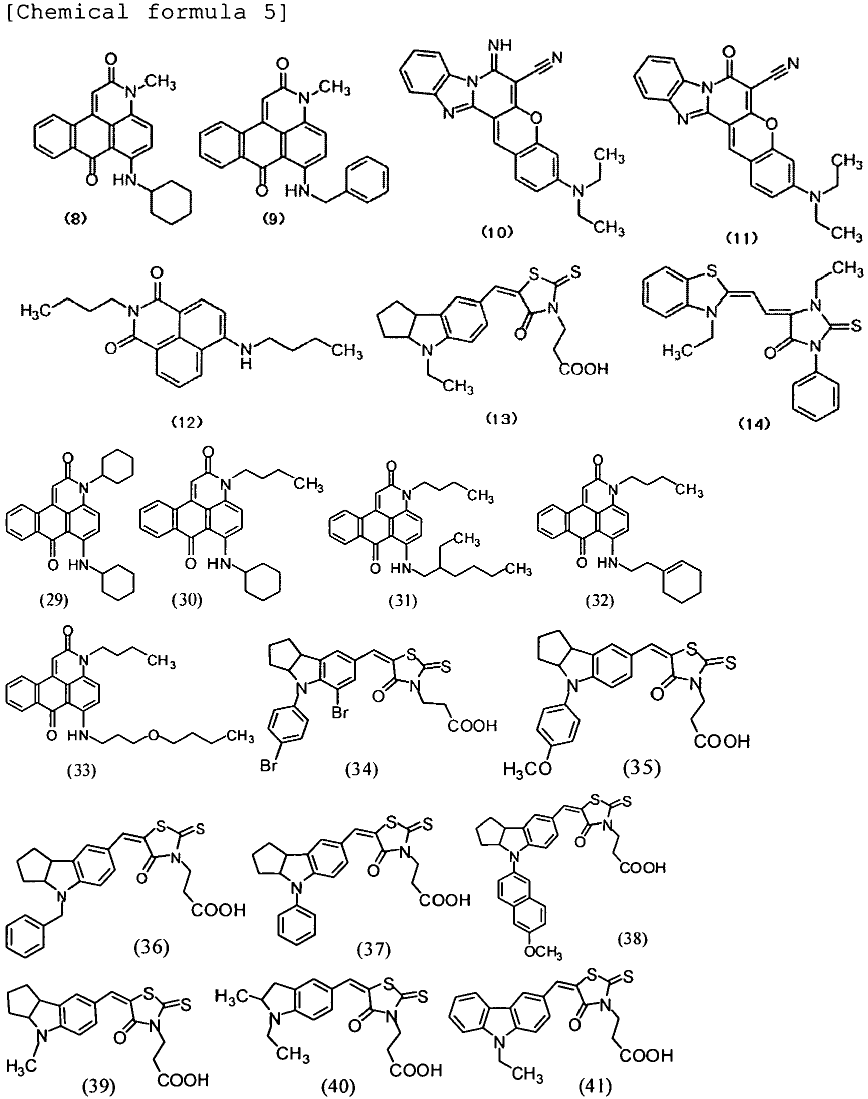

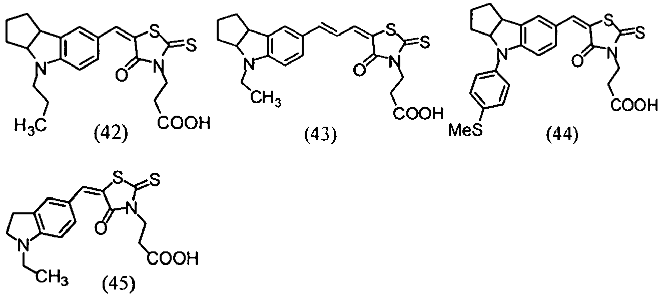

The general formula (7) will be described below.

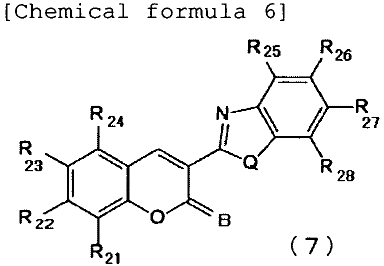

In the general formula (7), R₂₁ to R₂₄ each independently represent a hydrogen atom, an alkyl group, an alkoxy group, an amino group, or a halogen atom. R₂₁ and R₂₂, R₂₂ and R₂₃, or R₂₃ and R₂₄ may bind to each other to form a ring. R₂₅ to R₂₈ each independently represent a hydrogen atom, an alkyl group, an alkoxy group, or a halogen atom. B represents an oxygen atom or an NH group. Q represents an oxygen atom, a sulfur atom, and an N-R₂₉ group, wherein R₂₉ represents a hydrogen atom or an alkyl group.

No particular limitation is imposed on the alkyl group at R₂₁ to R₂₉ in the aforementioned general formula (7), and examples thereof include a methyl group, an ethyl group, a propyl group, and a butyl group. No particular limitation is imposed on the alkoxy group at R₂₁ to R₂₈, and examples thereof include a methoxy group, an ethoxy group, a propoxy group, and a butoxy group. No particular limitation is imposed on the amino group at R₂₁ to R₂₄, and examples thereof include an unsubstituted amino group; a mono-substituted amino group such as an N-methylamino group and an N-ethylamino group; and a di-substituted amino group such as an N,N-dimethylamino group, an N,N-diethylamino group, and an N,N-methylpropylamino group.

Examples of the halogen atom at R₂₁ to R₂₈ include a fluorine atom, a chlorine atom, a bromine atom, or an iodine atom. No particular limitation is imposed on the ring formed by binding of R₂₁ and R₂₂, R₂₂ and R₂₃, or R₂₃ and R₂₄, and examples thereof include an aromatic ring such as a benzene ring, a saturated ring such as a cyclohexane ring, a partially-saturated ring such as a cyclopentene ring, and a hetero ring such as a piperidine ring. Also, the ring may further contain a substituent as long as the substituent does not markedly deteriorate the preservation stability of the dye compound.

In the aforementioned general formula (7), particularly, when R₂₂ is an electron-donating substituent such as an amino group or an alkoxy group, the fluorescent intensity is increased. Thus, such a compound can be employed. When R₂₂ is a di-substituted amino group such as an N,N-dimethylamino group and an N,N-diethylamino group, a high fluorescent intensity is attained; therefore, such a compound can be employed. Q is an oxygen atom or a sulfur atom from the viewpoint of the labeling property. Particularly, when Q is an oxygen atom, a high fluorescent intensity is attained and a specific site can be effectively labeled; therefore, such a compound can be employed. When R₂₆ is an alkyl group such as methyl or a halogen atom such as a chlorine atom, a high fluorescent intensity is attained and a specific site can be effectively labeled; therefore, such a compound can be employed.

While a dye compound represented by the general formula (7) is commercially obtainable, it can also be synthesized in accordance with a publicly known method (for example, Dyes and pigments, Vol. 47 (Issues 1-2), pages 79-89 (2000)). Preferable specific examples of the dye compound represented by the general formula (7) (compounds (15) to (28)) will be shown below.

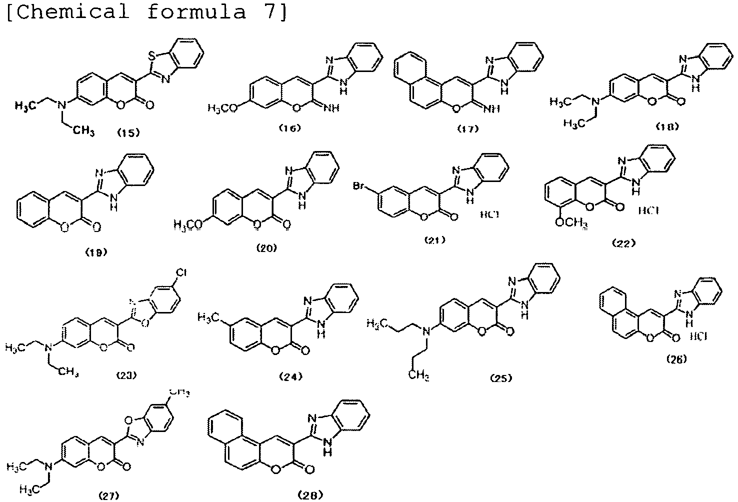

### (Compound)

A central nervous system tissue-labeling composition of the present invention is characterized by containing a compound capable of labeling at least one cell type present in a central nervous system tissue. The central nervous system tissue-labeling composition of the present invention can contain a compound selectively labeling at least one of optic nerve, optic tract, superior colliculus (optic tectum), pituitary gland, tectospinal (tectobulbar) tract, and reticular formation of the central nervous system tissue. In the present invention, "selectively labeling" refers to such a labeling property that at least a cell or a site clearly noted in the present invention is labeled while a tissue other than the aforementioned cell or site in the central nervous system tissue is not labeled, or the target cells or sites of the composition are labeled differently (labeled at a high or low level).

In consideration of migration of a compound represented by the general formula (1) encompassed by the central nervous system tissue-labeling compositions of the present invention into the target central nervous tissue, the compound can be a low molecular compound, and a compound having a molecular weight of 2000 or less is selected. Further, a compound having a molecular weight of 1000 or less, particularly 600 or less, can be employed.

Further, a compound of the present invention can be a fluorescent compound having a fluorescent property. Owing to a high sensitivity of a fluorescent compound, a low concentration of the compound is required for labeling, whereby the amount of the compound necessary for labeling can be relatively reduced. Also, selecting a combination of compounds with different labeling sites and fluorescent spectra enables multi-labeling. This is highly useful because more information can be obtained by single observation.

Because a central nervous system tissue-labeling composition of the present invention can migrate into the central nervous system tissue without being blocked by BBB or BCSFB, the composition can be administered without damaging the central nervous system tissue or the tissue connected to the central nervous system tissue in a living organism.

Therefore, according to a method for labeling the central nervous system tissue of a second embodiment of the present invention, a central nervous system tissue or a tissue connected to the central nervous system tissue can be labeled without being damaged. That is, a living organism can be labeled with the central nervous system tissue-labeling composition without causing surgical injury such as an incision in the tissue near the central nerve and a puncture in the central nervous system tissue or in the nervous tissue connected to the central nervous system tissue. It is to be noted that the present invention does not exclude the aforementioned labeling method involving surgical injury.

No particular limitation is imposed on the labeling method not causing surgical injury, and examples thereof include a method of exposing the central nervous system tissue-labeling composition to a living organism locally or systemically, a method by oral contact, a method by pulmonary contact, a method by nasal contact, a method by contacting with the digestive tract, a method by mucosal contact, a method by contacting with a body fluid, a method by sublingual contact, a method by intravascular contact such as contacting with the vein or artery, a method by intraperitoneal contact, an infusion method such as an intravaginal, subcutaneous, intradermal, intravesical, or intratracheal (intrabronchial) infusion, and a method of contacting with the living body by, for example, spraying or applying. When administering to the animal, the dosage form, administration route, and dose of the composition are appropriately selected depending on the weight and the condition of the subject animal.

A method for acquiring the information through visualization of the state of labeling according to a third embodiment of the present invention is characterized by labeling a central nervous system tissue in the living body with the central nervous system tissue-labeling composition to acquire an image. That is, the method is characterized by administering the central nervous system tissue-labeling composition of the present invention by any method, and a certain time later, irradiating the observation site with light of excitation wavelength, and measuring fluorescence of longer wavelength thus generated to create an image.

Examples of specific method of labeling of the present invention can include a method using probes such as a fluorescent probe and a radionuclide-labeled probe. Staining the central nervous system tissue with these probes enables imaging of the distribution and the orientation of a periphery nerve system tissue connected to the central nervous system tissue. In the present invention, staining the cell morphology of the central nervous system tissue refers to achieving a state in which at least one cell type present in the central nervous system tissue is stained so that the cell morphology of the cell type is clearly distinguished through, for example, the fluorescent color.

### (Observation method)

The observation method of the present invention is characterized by using a central nervous system tissue-labeling composition of the present invention. The measurement and the detection of the central nervous system tissue-labeling composition are carried out by a method publicly known to those skilled in the art. Although no particular limitation is imposed on the observation method employed in the present invention as long as the method does not affect central nervous system tissues, it is a method of capturing the state and the change of a biological sample as an image. Examples thereof include visible light observation, near-infrared light observation, infrared light observation, or laser microscopic observation, in which the eye tissue is irradiated with visible light, near-infrared light, or infrared light and then observed by a camera, CCD, etc., or fluorescent observation, fluorescence microscopic observation, fluorescence endoscopic observation, confocal fluorescence microscopic observation, multiphoton-excited fluorescence microscopic observation, narrow band imaging, in which, by using fluorescence endoscopy and so on, a biological sample is irradiated with excitation light from the excitation light source and the fluorescence emitting from the biological sample is observed, or optical coherence tomography (OCT), and further, observation under a soft X-ray microscope.

No particular limitation is imposed on the excitation wavelength used in the present invention, and the wavelength varies depending on the dye compound represented by the aforementioned general formula (1) used. No particular limitation is imposed on the excitation wavelength as long as it allows the dye compound represented by the aforementioned general formula (1) of the present invention to effectively emit fluorescence. The excitation wavelength is normally 200 to 1010 nm, or it can be 400 to 900 nm, and further, it can be 480 to 800 nm. When using the light in the near-infrared region, the wavelength of 600 to 1000 nm is normally employed, and the wavelength of 680 to 900 nm can be used since the light within such a range of wavelength has excellent permeability through the living body.

No particular limitation is imposed on the fluorescence excitation source used in the present invention, and various laser light sources can be used. Examples thereof include a dye laser, a semiconductor laser, an ion laser, a fiber laser, a halogen lamp, a xenon lamp, or a tungsten lamp. Furthermore, using various optical filters, a preferable excitation wavelength can be obtained or fluorescence only can be detected. As described above, the fluorescence is emitted inside the central nervous system tissue of an organism being irradiated with the excitation light, and imaging the central nervous system tissue, allowing easy detection of the light-emitting site. Alternatively, a bright-field image obtained by irradiation of visible light and a fluorescent image obtained by irradiation of excitation light can be combined by image processing to enable a more detailed observation of the central nervous system tissue. Further, a confocal microscope can be used for acquisition of an optical image of a section. Furthermore, a multiphoton-excited fluorescence microscope can be used for an observation of the inside of the tissue for its high accessibility to a deep part and spatial resolution.

### (Radiation labeling)

A central nervous system tissue-labeling composition of the present invention can also be used as a radionuclide-labeled probe. No particular limitation is imposed on the radionuclide type used for labeling, and it can be appropriately selected depending on the manner in which it is used. Specifically, for measurement by PET, a positron-emitting radionuclide such as ¹¹C, ¹⁴C, ¹³N, ¹⁵O, ¹⁸F, ¹⁹F, ⁶²Cu, ⁶⁸Ga, or ⁷⁸Br can be used. Among them, ¹¹C, ¹³N, ¹⁵O, or ¹⁸F can be used, of which ¹¹C or ¹⁸F can particularly be used. Also, for measurement by SPECT, a γ-emitting nuclide such as ⁹⁹mTc, ¹¹¹In, ⁶⁷Ga, ²⁰¹Tl, ¹²³I, or ¹³³Xe can be used. Among them, ⁹⁹mTc or ¹²³I can be used. Further, when measuring an animal other than a human, a radionuclide having a longer half-life such as ¹²⁵I can be used. For measurement by GREI, for example, ¹³¹I, ⁸⁵Sr, and ⁶⁵Zn can be used.

A central nervous system tissue-labeling composition labeled with a radionuclide can be imaged by, for example, autoradiography, positron emission tomography (PET) using a positron-emitting radionuclide, single photon emission computed tomography (SPECT) using various gamma-emitting nuclides. Also, the composition can be detected by magnetic resonance imaging (MRI) utilizing an MR signal originating from the fluorine nucleus and 13C. Further, the compound can also be imaged by a Compton camera (GREI), which is capable of multiple molecular simultaneous imaging as a next-generation molecular imaging apparatus. Also, a probe for the central nervous system tissue can be quantitated by using, for example, a liquid scintillation counter, an X-ray film, and an imaging plate.

Also, by measuring the concentration of the central nervous system tissue-labeling composition labeled with a radioisotope such as ¹⁴C in blood (or in urine, or in feces) by a method such as accelerator mass spectrometry (AMS), the pharmacokinetic information (such as area under the blood drug concentration time curve (AUC), the blood half-life (T1/2), the maximum blood concentration (Cmax), the time to maximum blood concentration (Tmax), the volume of distribution, the first pass effect, the bioavailability, and the rate of excretion in feces and urine) of an unmodified form or a metabolite of the labeled composition can be acquired.

The radionuclide may be contained in or bound to the compound represented by the general formula (1) or (7). No particular limitation is imposed on the method of labeling a radionuclide, and a method generally employed may be used. Also, a radionucleotide may substitute or be bound to at least a part of the elements constituting the compound represented by the general formula (1) or (7). When labeling the compound represented by the general formula (1) or (7) with a radionuclide, the resulting compound can have a radioactivity of approximately 1 to 100 µCi per 1 mM. In this case, no particular limitation is imposed on the dose of the central nervous system tissue-labeling composition as long as the composition does not affect the subject, and the dose is appropriately selected depending on the compound type and the radionuclide used for labeling.

### (Biological sample)

No particular limitation is imposed on the species in which the central nervous system tissue can be labeled with the central nervous system tissue-labeling compound of the present invention. Examples thereof include, as a vertebrate, teleosts such as Takifugu rubripes, Takifugu niphobles, Tetraodon nigroviridis, Oryzias latipes, and zebrafish, amphibians such as African clawed frogs, birds such as chickens and quails, small animals such as rats, mice, and hamsters, large animals such as goats, pigs, dogs, cats, cows, and horses, monkeys, chimpanzees, and humans. Particularly, the intraocular tissue of these organisms can be labeled alive. Also, as a biological sample, humans may be excluded.

Among these biological samples, zebrafish can be used. Zebrafish expresses Claudin-5 and Zonula Occludens-1, which are the major constituent protein of the tight junctions of BBB, in an embryo at three days post fertilization (3 dpf) (Brain Research Bulltein 75 (2008) 619-628). Major organs are formed on 6-7 dpf, and P-glycoprotein, which functions to excrete substances across BBB, is expressed by 8 dpf. Thus, zebrafish can be used for assessment of the central nervous system tissue. Further, there is such an advantage that, because zebrafish produces more than approximately 200 fertilized eggs per spawning, zebrafish having the identical genetic background can be obtained, which is convenient for screening.

### (Central nervous system tissue)

Examples of the central nervous system tissues that can be labeled with the central nervous system-labeling composition of the present invention include a central nervous system tissue composed of cerebrum (telencephalon), cerebral cortex, basal ganglia, midbrain, cerebellum, diencephalon, hindbrain (pallium), pons, medulla oblongata, spinal cord, optic tract, superior colliculus (optic tectum), pituitary gland, tectospinal (tectobulbar) tract, reticular formation, septal nuclei, amygdala, internal capsule, and optic nerve, these tissues in a pathological condition, or a neoplasm resulting from a disease and a cancer tissues. Also, when the central nervous system tissue other than the ones described above is present due to factors such as the organism type, the developmental stage, abnormal development, or diseases, such a tissue can also be encompassed. Particularly, a central nervous system-labeling composition of the present invention can label optic nerve, optic tract, superior colliculus (optic tectum), pituitary gland, tectospinal (tectobulbar) tract, and reticular formation.

No particular limitation is imposed on the cell types contained in the aforementioned central nervous system tissues. Examples thereof include neurons, oligodendrocytes, Schwann cells, Purkinje cells, amacrine cells, retinal ganglion cells, pyramidal cells, astrocytes, granule cells, glial cells, or tumor cells and undifferentiated cells (stem cells) thereof. Also, a central nervous system tissue-labeling composition of the present invention can label the cranial nerve such as the optic nerve. Staining the cranial nerve enables imaging of the distribution and the orientation of a periphery nerve system tissue connected to the central nervous system tissue. In the present invention, labeling a central nervous system tissue, namely labeling the cell morphology of the central nervous system tissue refers to achieving a state in which at least one cell type present in the central nervous system tissue is labeled so that the cell morphology of the cell type is clearly distinguished by an appropriate observation method.

### (Diagnosis of disease)

No particular limitation is imposed on a central nervous system disease to be diagnosed by imaging using the central nervous system tissue-labeling composition of the present invention. Examples thereof include Parkinson's disease, Alzheimer's disease, Huntington's disease, motor neuron disease, tauopathy, corticobasal degeneration, depression, epilepsy, migraine, spinocerebellar degeneration, brain tumor, cerebral hemorrhage, and cerebral infarction.

### (Preparation of the central nervous system tissue-labeling composition)

No particular limitation is imposed on the concentrations of the compound contained in a central nervous system tissue-labeling composition of the present invention as long as a central nervous system tissue can be detected, and it is appropriately adjusted depending on the target site and the compound used. The compound is normally used in a concentration of 0.001 ng/mL or more and 100 µg/mL or less. It can also be used in a concentration of 0.001 ng/mL or more and 10 µg/mL or less, and further, in a concentration of 0.001 ng/mL or more and 5 µg/mL or less.

A central nervous system tissue-labeling composition of the present invention is used by dissolving at least one of the dye compounds represented by the aforementioned general formula (1) or (7) in an appropriate solvent. No particular limitation is imposed on the solvent as long as it does not affect the living body. For example, a highly biocompatible aqueous liquid can be used. Specific examples thereof include water; physiological saline; a buffer such as phosphate buffered saline (PBS) and Tris; an alcohol solvent such as methanol, ethanol, isopropanol, butanol, ethyleneglycol, and glycerin; an organic solvent such as N,N-dimethylsulfoxide (hereinbelow, abbreviated as DMSO) and N,N-dimethylformamide (hereinbelow, abbreviated as DMF); a cell culture medium such as D-MEM and HBSS, or an infusion solution such as a lactate Ringer's solution. Particularly, these solvents can contain more than 50% water. Also, a mixture of two or more kinds of these solvents can be used.

No particular limitation is imposed on a production method of a central nervous system tissue-labeling composition of the present invention. For example, it may be produced by diluting a concentrated solution of the compound in the aforementioned solvent. A low water-soluble compound can be dissolved in an appropriate solvent first, and then dissolved in purified water for use. Particularly, methanol, ethanol, and DMSO can be used.

When controlling the salt concentration or pH to a suitable level for the living body is necessary, an additive or a combination of two or more of additives can be added to the central nervous system tissue-labeling composition of the present invention. No particular limitation is imposed on the additive used in the present invention as long as it does not affect the central nervous system tissue-labeling composition, and examples thereof include humectants, surface tension-preparing agents, viscosity enhancers, salts such as sodium chloride, various pH-preparing agents, pH buffers, antiseptics, antimicrobial agents, sweeteners, or flavoring agents.

The pH-preparing agent can be those that prepare pH to 5 to 9. No particular limitation is imposed on the pH-preparing agent, and examples thereof include hydrochloric acid, acetic acid, phosphoric acid, citric acid, malic acid, sodium hydroxide, or sodium hydrogen carbonate. Use of the central nervous system tissue-labeling composition of the present invention enables labeling of the central nervous system tissue without causing surgical injury such an incision in the tissue near the central nerve system and a puncture in the central nervous system tissue or in the nervous tissue connected to the central nervous system tissue.

A screening method according to a fourth embodiment of the present invention is characterized by detecting a compound acting on a central nervous system tissue in vivo by using the central nervous system tissue-labeling composition. The central nervous system tissue-labeling composition of the present invention labels the central nervous system tissue in an organism, for example zebrafish, which is a small teleost, alive. Using the composition's central nervous system-labeling property in a living organism, i.e., the composition's in vivo labeling property, as an index, the transferability of the compound-to-be-screened-for into the central nervous system tissue and the pharmacological effect of the compound-to-be-screened-for can be screened. Further, because live zebrafish, a living organism, is used, the safety of the compound-to-be-screened-for can be simultaneously screened.

Recently, zebrafish has been recognized as the third model animal after mice and rats in U.S. and U.K. It has been elucidated that the complete genome sequence of zebrafish has an 80% homology with that of humans, and also, zebrafish has nearly the same number of genes as humans, and the major organs, the development of tissues, and the structures are very similar between zebrafish and humans. Zebrafish can particularly be used for screening as a model animal because the process of differentiation and formation of each part (an organ and a part such as the heart, liver, kidney, and digestive tract) from a fertilized egg can be observed through a transparent body.

"Detecting a compound acting on a central nervous system tissue" refers to measuring, using a central nervous system tissue-labeling composition of the present invention, the change in the labeling property when a compound of interest (a compound-to-be-screened-for) is allowed to act on the central nervous system to detect the presence or absence and the characteristics of the compound acting on the central nervous system tissue. A specific example thereof is a screening method in which a compound-to-be-screened-for and the central nervous system tissue-labeling composition of the present invention are contacted with zebrafish to observe the effect of the presence of the compound-to-be-screened-for on the condition of the labeling of the central nervous system tissue with the central nervous system tissue-labeling composition.

No particular limitation is imposed on the method for contacting the compound-to-be-screened-for. When the compound-to-be-screened-for is water-soluble, a method of administering the compound-to-be-screened-for into the rearing water may be employed. When the compound-to-be-screened-for is water-insoluble, methods such as singly administering the compound-to-be-screened-for by dispersing it into the rearing water, administering it with a trace amount of surfactants and DMSO, orally administering it by mixing with the feed for zebrafish, or parenterally administering it by an injection may be employed. Of these, a method of administering the compound-to-be-screened-for into the rearing water can be employed for easiness.

Using one or more of the central nervous system tissue-labeling compositions of the present invention as an active ingredient, the effect, side effect, or safety of the compound-to-be-screened-for on the central nervous system tissue in an organism can be screened for. That is, the effect of the compound-to-be-screened-for on an organism can be screened in vivo using, for example, zebrafish. The central nervous system tissue-labeling composition used can be selected as desired depending on the target site, purpose, examination measures, etc. Further, owing to the labeling property of the central nervous system tissue-labeling composition, the application of the composition is expected to be expanded to, for example, the development of highly accurate diagnosis and treatment method of a disease. Thus, the central nervous system tissue-labeling composition can be used as a diagnostic composition.

The aforementioned compound-to-be-screened-for refers to the generic term for compounds having chemical actions. No particular limitation is imposed on the compound, and examples thereof include pharmaceutical products, organic compounds, therapeutic agents, investigational new drugs, agricultural chemicals, cosmetics, environmental pollution substances, or endocrine disrupting substances. Depending on the purpose of screening, zebrafish is not limited to wild zebrafish, and various zebrafish disease models can be used. When using a disease model, the effect of a new drug candidate compound is found out through observation, which can then be applied to screening of a therapeutic or preventive drug for a disease.

Also, small teleosts can be employed in the screening method of the present invention. No particular limitation is imposed on the small teleost used in the screening method of the present invention, and examples thereof include zebrafish, pufferfish, goldfish, Oryzias latipes, and giant rerio. Small teleosts can be used since they are highly excellent in terms of speed and cost compared to mice and rats. Particularly, zebrafish can be used because the genome of the organism has been almost completely sequenced, and it can be easily reared and bred, and distributed at low cost, and further, the basic structures of the major organs and tissues are formed within 48 to 72 hours after fertilization.

### (Intraoperative diagnosis)

A central nervous system tissue-labeling composition of the present invention can be used, for example, for site-specifically and selectively labeling a cellular tissue at a pathological site and a region that is presumed to be tumor during brain surgery so as to distinguish those tissue and region from a normal cell, or for observing the change in the tissue caused by a disease. As an observation tool, a cerebral endoscope (fiberscope) and a microscope for brain surgery can be used. The central nervous system tissue-labeling composition of the present invention can label the central nervous system tissue in a living organism without requiring highly invasive operations such as exposing the central nervous system tissue and infusing a labeling agent into the central nervous system tissue or the tissue connected to the central nervous system tissue. Accordingly, utilizing the aforementioned discriminative ability of the central nervous system tissue-labeling composition, the composition can be applied as a diagnostic agent. Although no particular limitation is imposed on the diagnostic agent, the compound can be used as, for example, a diagnostic agent for examination of the brain function and for a brain disease.

### (Brain function imaging and mapping)

A central nervous system tissue-labeling composition of the present invention can be used as a probe for brain function imaging and mapping. The fluorescent characteristics of the central nervous system tissue-labeling composition of the present invention vary depending on the biomolecules to be interacting with and the environment of a solvent. Thus, by detecting a change in the fluorescent characteristics, a change in the state of activity of the brain neurons can be detected.

### (Sensitizer (photodynamic therapy))

A central nervous system tissue-labeling composition of the present invention can also be used as a photosensitizer. A photosensitizer is a chemical compound that is activated upon irradiation with photoactivating light and converted into a cytotoxic form, thereby killing the target cell or attenuating the proliferation ability of the target cell.

### (Extrapolation to humans)

A central nervous system tissue-labeling composition of the present invention can also be applied to humans. The extrapolation to humans can be confirmed by the general approximation based on the recognition of similarities and differences between the central nervous system tissues of humans and those of the experimental animals. Although some examples will be shown below, the confirmation of the extrapolation to humans is not limited thereto.
(1) Labeling a central nervous system tissue of humans and that of non-human live biological samples to confirm similarities. Examples of the non-human live biological sample include mammals such as mice, hamsters, rats, guinea pigs, rabbits, dogs, pigs, cats, and monkeys, and teleosts such as zebrafish.
(2) Confirming the central nervous system tissue-labeling property in a fixed tissue section of the aforementioned non-human live biological sample and confirming that a similar labeling property to that obtained in a live biological sample is observed.
(3) Confirming the central nervous system tissue-labeling property in a fixed tissue sample of humans.

By confirming the aforementioned three points, the central nervous system tissue-labeling composition of the present invention can be confirmed to be applicable also to humans. As another method, the extrapolation to humans can be verified by administering an infinitesimal amount of radiolabeled central nervous system tissue-labeling composition of the present invention to the human body and confirming the localization of the composition to the central nervous system tissue. This technique is called microdosing test. Further, as an alternative method, the following method can be employed: (1) identifying the target biomolecule or mechanism of labeling of a central nervous system tissue-labeling composition of the present invention in a central nervous system tissue of a non-human biological sample, (2) identifying a biomolecule or mechanism of labeling in humans homologous to the aforementioned target biomolecule or molecular mechanism of labeling, (3) introducing the biomolecule or mechanism of labeling in humans into a non-human experimental animal by genetic modification, and (4) using the experimental animal thus obtained, confirming that labeling is achieved via the biomolecule or mechanism of labeling thus introduced.

As the non-human biological sample, particularly zebrafish can be used. The blood-brain barrier (BBB), which is an important function in the central nervous system tissue, is operative also in zebrafish similarly to a number of other vertebrates. Use of zebrafish is highly advantageous as the cost of rearing is low compared to other organisms such as mice, and only a small amount of the compound is required. Further, not only morphological models but also models of a number of human diseases have been produced. In view of the foregoing, zebrafish can be used for confirmation of the extrapolation of the central nervous system tissue-labeling composition of the present invention to humans.

### Examples

Hereinbelow, the present invention will be described in more detail with reference to Examples. However, these Examples serve as specific examples for deeper understanding of the present invention, and the present invention is not limited to these specific examples in any way. Also, unless otherwise specifically noted, "%" is the mass standard. Further, analytical apparatuses used are ¹H nuclear magnetic resonance spectrometric analysis (ECA-400, manufactured by JEOL Ltd.), LC/TOF MS (LC/MSD TOF, manufactured by Agilent Technologies, Inc.), and a multispectral microplate reader (Varioskan Flash, manufactured by Thermo Fisher Scientific Inc.). While the dye compounds represented by the general formulas (1) to (7) are commercially obtainable, they can also be synthesized in accordance with a publicly known method.

### Example 1

Labeling the central nervous system tissue with the central nervous system tissue-labeling composition Distilled water was added to a 1 mg/mL solution of the aforementioned compound (8) in DMSO to prepare a labeling solution 1 having a concentration of the aforementioned compound (8) of 1 µg/mL. Into an arbitrary well of a 24-well multiplate (manufacture by IWAKI), 1 mL of the labeling solution 1 and a day-7 embryo (7 dpf) of juvenile zebrafish were placed, and the plate was left to stand for one hour. Subsequently, the labeling solution 1 in the well was removed and replaced by 1 mL of distilled water. This operation was repeated three times. Then, juvenile zebrafish was removed from the well and embedded in 5% low melting point agarose gel on a slide glass so as to restrict movement, and the state of labeling in the central nervous system tissues were observed from the lateral side of zebrafish by a fluorescence stereomicroscope (manufactured by Leica Microsystems, MZ16FA). Also, the brain nerve tissues were observed from the parietal region by a confocal microscope (manufactured by Carl Zeiss, Inc., Pascal Exciter). As a result, fluorescence was observed in the brain nerve tissues of zebrafish. The state of labeling in the brain varied depending on the site, and it was observed that optic nerve, optic tract, superior colliculus (optic tectum), pituitary gland, tectospinal (tectobulbar) tract, and reticular formation were intensely labeled.

### Examples 2 to 7

Zebrafish was labeled and observed by similar operations to Example 1 except for changing the dye compound (8) of Example 1 to the dye compounds (9) to (14) listed in Table 1 and for using labeling solutions 2 to 7. Compounds (10) to (14) are not in the scope of the present claims.

### Comparative Example 1

Zebrafish was labeled and observed by similar operations to Example 1 except for changing the dye compound (8) of Example 1 to fluorescein.

The labeling properties (++: a central nervous system tissue(s) is intensely labeled, +: a central nervous system tissue(s) is weakly labeled, and -: not labeled) were assessed in the aforementioned Examples 1 to 7 and in Comparative Example 1. The results thus obtained are shown in Table 1. It is to be noted that the excitation wavelength and the fluorescence wavelength of the dye compounds of Examples 1 to 7 and Comparative Example 1 were obtained by measuring an aqueous solution prepared by diluting a 10 mg/mL DMSO solution 500-fold with distilled water by FL4500 fluorescence spectrophotometer, Hitachi High-Technologies Corporation.

### [Table 1]

**Table 1**

| Example No. | Compound No. | λex | λem | Labeling property |
|---|---|---|---|---|
| 1 | (8) | 599 | 619 | ++ |
| 2 | (9) | 480 | 556 | ++ |
| 3 | (10) | 556 | 577 | ++ |
| 4 | (11) | 555 | 576 | + |
| 5 | (12) | 466 | 551 | ++ |
| 6 | (13) | 576 | 604 | ++ |
| 7 | (14) | 530 | 560 | ++ |
| Comparative Example No. | Compound | λex | λem | Labeling property |
| 1 | Fluorescein | 494 | 521 | - |

### Examples 8 and 9 (not in the scope of the claims)

Zebrafish was labeled and observed by similar operations to Examples 6 and 7 except for changing a day-7 embryo (7 dpf) of juvenile zebrafish to a day-14 embryo (14 dpf) of juvenile zebrafish. As a result, fluorescence was observed also in the brain nerve tissues of 14 dpf juvenile zebrafish. The state of labeling in the brain varied depending on the site, and it was observed that optic nerve, optic tract, superior colliculus (optic tectum), pituitary gland, tectospinal (tectobulbar) tract, and reticular formation were intensely labeled.

### Example 10 (not in the scope of the claims)

A 3-month-old B10 mouse was sacrificed by diethyl ether anesthesia, and the brain is collected. The brain thus removed is embedded in an OCT compound, and then frozen in isopentane cooled with liquid nitrogen. The resulting brain was sliced into thin sections of approximately 10 µm in thickness in a cryostat cooled to -20°C. The thin sections were then placed on a slide glass and dried, whereby a section of the brain tissue was prepared. To the section of ocular tissue thus prepared, a 1 ug/mL solution of the compound (13) in PBS was added, followed by incubation for one hour. After one hour, the slide glass was washed with PBST (PBS containing 0.2% Triton-X100) three times, and then sealed with a cover glass. Upon observation of the slide glass by a confocal microscope (manufactured by Carl Zeiss, Inc., Pascal Exciter), the compound (13) was confirmed to exert a labeling property in a mouse brain tissue section.

### Example 11 (not in the scope of the claims)

The compound (13) is added to an equimolar solution of NaOH so that a concentration of 10 mg/ml is reached, and the resulting mixture is centrifuged at 14 krpm for five minutes to obtain a supernatant. Then, 0.2 ml of the supernatant thus obtained is intraperitoneally administered to a 3-month-old B10 mouse in a single dose. After one hour, the animal thus treated is sacrificed by diethyl ether anesthesia, and the brain is collected. The brain thus removed is embedded in an OCT compound, and then frozen in isopentane cooled with liquid nitrogen. The resulting brain was sliced into thin sections of approximately 10 µm in thickness in a cryostat cooled to -20°C. The thin sections were then placed on a slide glass and dried, whereby a section of the brain tissue was prepared. The brain tissue section thus prepared was observed under a confocal microscope (manufactured by Carl Zeiss, Inc., Pascal Exciter). As a result, the compound was confirmed to exert a labeling property in a mouse brain by intraperitoneal administration.

### Reference Example 1

Zebrafish was labeled and observed by similar operations to Comparative Example 1 except for changing 7 dpf juvenile zebrafish used in Comparative Example 1 to 3 dpf juvenile zebrafish. As a result, the brain nerve tissues were not observed to be stained either in the 3 dpf juvenile zebrafish.

Typical synthesis examples 1 and 2 of the compound of the general formula (7) will then be described.

### (Synthesis Example 1)

Synthesis of the aforementioned compound (16):
Into a solution of 6.0 g (39 mmol) of 2-hydroxy-4-methoxybenzaldehyde in 70 mL of acetonitrile, 6 g (38 mmol) of (2-benzimidazoyl)acetonitrile, 0.3 g (3.5 mmol) of piperidine, and 0.2 g (3.3 mmol) of acetic acid were added, followed by stirring for eight hours while heating the mixture to reflux. Upon completion of the reaction, 50 mL of water was slowly added dropwise while cooling. The mixture was cooled to room temperature to precipitate an individual, which was collected by filtration and washed with a mixture of acetonitrile 50 mL/water 100 mL to give 10.5 g (yield 98.7%) of the compound (16). The compound was confirmed to be the objective substance by the aforementioned analytical apparatuses.

### Synthesis Example 2

Synthesis of the aforementioned compound (20):
Into a solution of 1.0 g (3.4 mmol) of the aforementioned compound (16) in 20 mL of ethanol, a mixture of concentrated hydrochloric acid 4 mL/water 4 mL was added dropwise, followed by stirring for four hours while heating the mixture to reflux. Upon completion of the reaction, the resulting mixture was cooled to precipitate a solid. The solid was collected by filtration and washed with ethanol to give 1.0 g of hydrochloride of compound (20). Further, 0.88 g of the hydrochloride thus obtained was dissolved in chloroform and the resulting mixture was neutralized with sodium carbonate. The mixture thus obtained was separated, and the resulting chloroform layer was concentrated under reduced pressure to give 0.47 g (yield from the hydrochloride 49%) of the compound (20). The compound was confirmed to be the objective substance by the aforementioned analytical apparatuses.

### Examples 12 to 25 (not in the scope of the claims)

Zebrafish was labeled and observed by similar operations to Example 1 except for changing the dye compound (8) of Example 1 to the dye compounds (15) to (28) listed in Table 2 and for using labeling solutions 12 to 25. As a result, fluorescence was observed also in the brain nerve tissues of 14 dpf juvenile zebrafish. The state of labeling in the brain varied depending on the site, and it was observed that optic nerve, optic tract, superior colliculus (optic tectum), pituitary gland, tectospinal (tectobulbar) tract, and reticular formation were intensely labeled.

The labeling properties (++: a central nervous system tissue(s) is intensely labeled, +: a central nervous system tissue(s) is weakly labeled, and -: not labeled) and the fluorescence sensitivities (++: a central nervous system tissue(s) is intensely observed, +: a central nervous system tissue(s) is weakly observed, and -: not labeled) were assessed in the aforementioned Examples 12 to 25 and in Comparative Example 1.

The results thus obtained are shown in Table 2. It is to be noted that the excitation wavelength and the fluorescence wavelength of the dye compounds of Examples 12 to 25 and Comparative Example 1 were obtained by measuring an aqueous solution prepared by diluting a 10 mg/mL DMSO solution 500-fold with distilled water by FL4500 fluorescence spectrophotometer, Hitachi High-Technologies Corporation.

### [Table 2]

**Table 2**

| Example No. | Compound No. | Excitation wavelength λex | Fluorescence wavelength λem | Stokes shift λex-λem | Labeling property | Fluorescence sensitivity |
|---|---|---|---|---|---|---|
| Example 12 | 15 | 469 | 555 | 86 | ++ | ++ |
| Example 13 | 16 | 380 | 470 | 90 | + | + |
| Example 14 | 17 | 410 | 490 | 80 | + | + |
| Example 15 | 18 | 464 | 514 | 50 | ++ | ++ |
| Example 16 | 19 | 459 | 520 | 62 | ++ | ++ |
| Example 17 | 20 | 380 | 470 | 90 | + | + |
| Example 18 | 21 | 360 | 490 | 130 | + | + |
| Example 19 | 22 | 360 | 510 | 150 | + | + |
| Example 20 | 23 | 422 | 476 | 54 | ++ | + |
| Example 21 | 24 | 380 | 490 | 110 | + | + |
| Example 22 | 25 | 463 | 509 | 46 | ++ | ++ |
| Example 23 | 26 | 410 | 540 | 130 | + | + |
| Example 24 | 27 | 472 | 504 | 32 | ++ | ++ |
| Example 25 | 28 | 410 | 540 | 130 | + | + |
| Comparative Example 01 | Fluorescein | 494 | 521 | 27 | Absence | Absence |

### Example 26 (not in the scope of the claims)

Into a 100 mL beaker, 30 mL of the labeling solution 20 prepared in Example 20 was poured. Then, 3-month-old zebrafish was placed and left there for one hour. Subsequently, the labeling solution 20 was removed and replaced by 50 mL of distilled water. This operation was repeated three times. Then, zebrafish was fixed in a phosphate buffer containing 4% paraformaldehyde and then embedded in 5% low melting point agarose gel. Using a linear slicer PRO7 (manufactured by Dosaka EM Co., Ltd.), a specimen of exposed brain was prepared. Upon observation of the specimen thus prepared under a confocal microscope (manufactured by Carl Zeiss, Inc., Pascal Exciter), the central nervous system tissue was observed to be stained also in 3-month-old adult zebrafish, based on which the labeling solution 20 was confirmed to exert a labeling property on the central nervous system tissues also in an organism in which the blood-brain barrier is operative.

### Example 27 (not in the scope of the claims)

A 3-month-old B10 mouse was sacrificed by diethyl ether anesthesia, and the brain is collected. The brain thus removed is embedded in an OCT compound, and then frozen in isopentane cooled with liquid nitrogen. The resulting brain was sliced into thin sections of approximately 10 µm in thickness in a cryostat cooled to -20°C. The thin sections were then placed on a slide glass and dried, whereby a section of the brain tissue was prepared. To the section of ocular tissue thus prepared, a 1 ug/mL solution of the compound (27) in PBS was added, followed by incubation for one hour. After one hour, the slide glass was washed with PBST (PBS containing 0.2% Triton-X100) three times, and then sealed with a cover glass. Upon observation of the slide glass under a confocal microscope (manufactured by Carl Zeiss, Inc., Pascal Exciter), the compound (27) was confirmed to exert a labeling property in a mouse brain tissue section.

### Example 28 (not in the scope of the claims)

The compound (27) was dissolved in chloroform, to which concentrated hydrochloric acid was added while stirring to form a precipitate. The precipitate was collected by filtration under reduced pressure. The precipitate thus collected was dried in a vacuum oven at 50°C for 24 hours to give hydrochloride of the compound (27). The hydrochloride of compound 27 is dissolved in PBS so that a concentration of 1 mg/mL is reached, and 0.2 ml of this solution is intraperitoneally administered to a 3-month-old B10 mouse in a single dose. After one hour, the animal thus treated is sacrificed by diethyl ether anesthesia, and the brain is collected. The brain thus removed is embedded in an OCT compound, and then frozen in isopentane cooled with liquid nitrogen. The resulting brain was sliced into thin sections of approximately 10 µm in thickness in a cryostat cooled to -20°C. The thin sections were then placed on a slide glass and dried, whereby a section of the brain tissue was prepared. The brain tissue section thus prepared was observed under a confocal microscope (manufactured by Carl Zeiss, Inc., Pascal Exciter). As a result, the compound was confirmed to exert a labeling property in a mouse brain by intraperitoneal administration.

### Example 2 9 (not in the scope of the claims)

Juvenile zebrafish was labeled by the same operations as Example 27 and then fixed in 4% PFA, and subsequently embedded in 5% low melting point agarose gel. Using a linear slicer Pro7 (manufactured by Dosaka EM Co., Ltd.), thin sections of the zebrafish were prepared, which were mounted on a slide glass. Upon observation of the section thus prepared under a confocal microscope (manufactured by Carl Zeiss, Inc., Pascal Exciter), particularly optic tectum and reticular formation of the brain of the zebrafish were confirmed to be intensely labeled.

It should be noted that Patent Literature 3 discloses a method for screening compounds for the central nervous system using zebrafish. According to this literature, it is described that because the expression of BBB transporter gene is incomplete in juvenile zebrafish, migration of dyes administered, namely Evans blue, fluorescein, and rhodamine 123, into the brain are confirmed up to 4 dpf, 8 dpf, and 5 dpf, respectively; however, because BBB is formed by 10 dpf, no dye will be observed to migrate into the brain any longer.

However, in the study conducted by the present inventors, as a result of an attempt to confirm the stainability of fluorescein in 3 dpf zebrafish, no stainability was observed (Reference Example 1). Meanwhile, the central nervous system tissue-labeling compositions of the present invention is able to stain the brain tissue in both 14 dpf zebrafish (Examples 8 and 9) and 3-month-old zebrafish (Example 26), and the state of staining in zebrafish in these Examples is similar to that observed in 7 dpf zebrafish. From these results, it is understood that BBB is already operative in zebrafish used by the present inventors as of 3 dpf, and regardless of the fact that BBB is fully formed (after 14 dpf), the central nervous system tissue-labeling compositions of the present invention is still able to label the central nervous system tissue.

### Examples 30 to 46

### (Examples 35 to 46 not in the scope of the claims)

Zebrafish was labeled and observed by similar operations to Example 1 except for changing the dye compound (8) of Example 1 to the dye compounds (29) to (45) listed in Table 3 and for using labeling solutions 29 to 45. It is to be noted that only the labeling solution 45 used in Example 46 had a concentration of the dye compound of 3 µg/mL. As a result, fluorescence was observed in the brain nerve tissues of 7 dpf juvenile zebrafish. The state of labeling in the brain varied depending on the site, and it was observed that optic nerve, optic tract, superior colliculus (optic tectum), pituitary gland, tectospinal (tectobulbar) tract, and reticular formation were intensely labeled. The labeling properties (++: a central nervous system tissue(s) is intensely labeled, +: a central nervous system tissue(s) is weakly labeled, and -: not labeled) were assessed in the aforementioned Examples 30 to 46. The results thus obtained are shown in Table 3. It is to be noted that the excitation wavelength and the fluorescence wavelength of the dye compounds were obtained by measuring 5 µM chloroform solutions of the compounds in Examples 30 to 34, and 5 µM DMSO solutions of the compounds in Examples 35 to 46 by FL4500 fluorescence spectrophotometer, Hitachi High-Technologies Corporation.

### [Table 3]

**Table 3**

| Example No. | Compound No. | Excitation wavelength λex | Fluorescence wavelength λem | Fluorescence sensitivity |
|---|---|---|---|---|
| 30 | 29 | 547 | 592 | + |
| 31 | 30 | 548 | 575 | + |
| 32 | 31 | 547 | 574 | + |
| 33 | 32 | 544 | 573 | + |
| 34 | 33 | 544 | 573 | + |
| 35 | 34 | 474 | 590 | + |
| 36 | 35 | 521 | 634 | ++ |
| 37 | 36 | 510 | 584 | + |
| 38 | 37 | 502 | 604 | ++ |
| 39 | 38 | 521 | 646 | + |
| 40 | 39 | 517 | 590 | ++ |
| 41 | 40 | 509 | 593 | ++ |
| 42 | 41 | 452 | 540 | + |
| 43 | 42 | 519 | 592 | ++ |
| 44 | 43 | 564 | 671 | ++ |
| 45 | 44 | 518 | 647 | ++ |
| 46 | 45 | 510 | 597 | + |

### Industrial Applicability

The present invention provides a central nervous system tissue-labeling composition capable of labeling a central nervous system tissue in a live biological sample and imaging the cell morphology of the central nervous system tissue with high sensitivity. Hence, the central nervous system tissue-labeling composition serves as a necessary material for a research in the area of the central nervous system and a technology pertaining to imaging of the central nervous system tissue. Also, in the development and discovery of drugs associated with central nervous system diseases, the central nervous system tissue-labeling composition allows chronological assessment of the central nervous system tissue, enabling highly accurate screening with high throughput at low cost. This dramatically progresses the development of new diagnostic and therapeutic methods for a disease, and further, expands research on the central nervous system, establishing a highly effective basic technology for not only industrial but also practical applications.

This application claims priority to Japanese patent application No. 2009-296270, filed December 25, 2009, the content of which can be used as further reference.

## Claims

1. A central nervous system tissue-labeling composition for use in the diagnosis of a central nervous system disease in vivo comprising, as an active ingredient, a compound represented by a general formula (1) 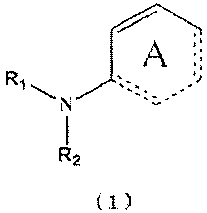 wherein, in the general formula (1), R₁ to R₂ each independently represent a hydrogen atom, an alkyl group, an aralkyl group, or an aryl group, and an aromatic ring A represents a skeletal structure represented by the following general formula (2): 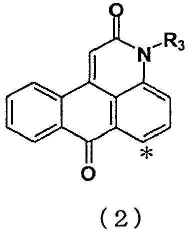 wherein, in the general formula (2), R₃ represents a hydrogen atom, an alkyl group, an aralkyl group, or an aryl group, and '*' represents a binding site to N in the general formula (1).

2. The central nervous system tissue-labeling composition for use according to Claim 1, wherein the compound is able to label at least any one of optic nerve, optic tract, superior colliculus (optic tectum), pituitary gland, tectospinal (tectobulbar) tract, and reticular formation.

3. The central nervous system tissue-labeling composition for use according to Claim 1 or 2, wherein the compound is a fluorescent compound.

4. The central nervous system tissue-labeling composition for use according to any one of Claims 1 to 3, wherein the compound is labeled with a radionuclide.

5. A method for labeling the central nervous system tissue comprising using the central nervous system tissue-labeling composition according to any one of Claims 1 to 4 to label a central nervous system tissue in a living body, wherein the method is not a method for treatment of the human or animal body by surgery.

6. A screening method comprising using the central nervous system tissue-labeling composition according to any one of Claims 1 to 4 to detect a compound acting on a central nervous system tissue in vivo, wherein the method is not a method for treatment of the human or animal body by surgery.

## Patentansprüche

1. Zusammensetzung zur Markierung von Gewebe des Zentralnervensystems zur Verwendung bei der in-vivo-Diagnose einer Zentralnervensystemkrankheit, die als aktive Komponente eine durch die allgemeine Formel (1) 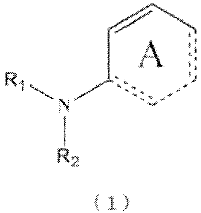 dargestellte Verbindung umfasst,
wobei in der Formel (1) R₁ bis R₂ jeweils unabhängig voneinander ein Wasserstoffatom, eine Alkylgruppe, eine Aralkylgruppe, oder eine Arylgruppe darstellen und ein aromatischer Ring A eine Skelettstruktur darstellt, welche durch die folgende allgemeine Formel (2) dargestellt wird: 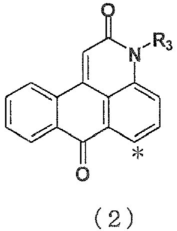 wobei in der allgemeinen Formel (2) R₃ ein Wasserstoffatom, eine Alkylgruppe, eine Aralkylgruppe oder eine Arylgruppe darstellt und "*" eine Bindungsstelle zu N in der allgemeinen Formel (1) darstellt.

2. Zusammensetzung zur Markierung von Gewebe des Zentralnervensystems zur Verwendung nach Anspruch 1, wobei die Verbindung in der Lage ist, mindestens den Sehnerv, die Sehbahn, das Colliculus superior (Tectum opticum), die Hypophyse, den Tektospinaltrakt (tektobulbaren Trakt) und/oder die Retikulärformation zu markieren.

3. Zusammensetzung zur Markierung von Gewebe des Zentralnervensystems zur Verwendung nach Anspruch 1 oder 2, wobei die Verbindung eine fluoreszierende Verbindung ist.

4. Zusammensetzung zur Markierung von Gewebe des Zentralnervensystems zur Verwendung nach einem der Ansprüche 1 bis 3, wobei die Verbindung mit einem Radionuklid markiert ist.

5. Verfahren zur Markierung des Gewebes des Zentralnervensystems, umfassend die Verwendung der Zusammensetzung zur Markierung von Gewebe des Zentralnervensystems nach einem der Ansprüche 1 bis 4 zur Markierung eines Gewebes eines Zentralnervensystems in einem lebenden Körper, wobei das Verfahren kein Verfahren zur chirurgischen Behandlung des menschlichen oder tierischen Körpers ist.

6. Screening-Verfahren, umfassend die Verwendung der Zusammensetzung zur Markierung von Gewebe des Zentralnervensystems nach einem der Ansprüche 1 bis 4 zum Nachweis einer Verbindung, die in vivo auf ein Gewebe eines Zentralnervensystems wirkt, wobei das Verfahren kein Verfahren zur chirurgischen Behandlung des menschlichen oder tierischen Körpers ist.

## Revendications

1. Composition de marquage d'un tissu du système nerveux central destinée à être utilisée dans le diagnostic d'une maladie du système nerveux central in vivo comprenant, comme ingrédient actif, un composé représenté par la formule générale (1) 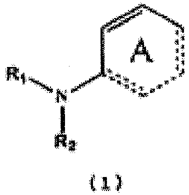 dans laquelle, dans la formule générale (1), R₁ et R₂ représentent chacun indépendamment un atome d'hydrogène, un groupe alkyle, un groupe aralkyle ou un groupe aryle, et un noyau aromatique A représente une structure squelettique représentée par la formule générale (2) 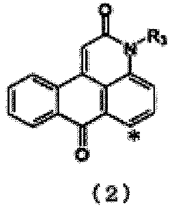 dans laquelle, dans la formule générale (2), R₃ représente un atome d'hydrogène, un groupe alkyle, un groupe aralkyle ou un groupe aryle, et '*' représente un site de liaison à N dans la formule générale (1).

2. Composition de marquage d'un tissu du système nerveux central destinée à être utilisée selon la revendication 1, dans laquelle le composé est capable de marquer au moins l'un quelconque d'un nerf optique, du tractus optique, du colliculus supérieur (tectum optique), de la glande pituitaire, du tractus tecto-spinal (tecto-bulbaire) et d'une formation réticulaire.

3. Composition de marquage d'un tissu du système nerveux central destinée à être utilisée selon la revendication 1 ou 2, dans laquelle le composé est un composé fluorescent.

4. Composition de marquage d'un tissu du système nerveux central destinée à être utilisée selon l'une quelconque des revendications 1 à 3, dans laquelle le composé est marqué avec un radionucléide.

5. Procédé de marquage d'un tissu du système nerveux central comprenant l'utilisation de la composition de marquage d'un tissu de système nerveux central selon l'une quelconque des revendications 1 à 4 pour marquer un tissu du système nerveux central dans un corps vivant, où le procédé n'est pas un procédé pour le traitement du corps humain ou animal par chirurgie.

6. Procédé de criblage comprenant l'utilisation de la composition de marquage d'un tissu du système nerveux central selon l'une quelconque des revendications 1 à 4 pour détecter un composé agissant sur un tissu du système nerveux central in vivo, où le procédé n'est pas un procédé pour le traitement du corps humain ou animal par chirurgie.
